# EUROPEAN PATENT SPECIFICATION

(11) **EP 0 508 430 B1**
(45) Date of publication and mention of the grant of the patent: **17.02.1999**
(21) Application number: 92106177.6
(22) Date of filing: 09.04.1992
(51) Int. Cl.: C07D 263/04

(54) **Process for the preparation of bisoxazolidines containing urethane groups**
Verfahren zur Herstellung von Urethangruppen enthaltende Bisoxazolidinen
Procédé de préparation de bisoxazolidines contenant des groupes urethanes

(30) Priority: 10.04.1991 IT MI910987
(43) Date of publication of application: 14.10.1992
(73) Proprietor: ENICHEM S.p.A., 20124 Milano (IT)
(72) Inventor: Greco, Alberto, Dr., I-20077 Dresano, Milan (IT); Mizia, Franco, Dr., I-20097 San Donato Milanese, Milan (IT); Rivetti, Franco, Dr., I-20139 Milan (IT)
(74) Representative: Weinhold, Peter, Dr.

(56) References cited:
- FR-A- 2 286 134
- Methoden der Organischen Chemie, Houben-Weyl, E4, 1983, pages 168-9

## Description

The present invention relates to a process for the preparation of polyoxazolidines containing urethane groups. These polyoxazolidines are useful as latent cross-linking agents in moisture curing systems based on polyisocyanates, acrylate polymers and polyepoxides in coating, sealant and adhesive compositions.

US-A-3,743,626 describes the use of some polyoxazolidines as hardeners, under atmospheric humidity and temperature conditions, for adhesives based on both aromatic and aliphatic polyisocyanates. As it is described in US-A-4,138,545, these polyoxazolidines can be obtained by means of the reaction of an oxazolidine of formula (A): 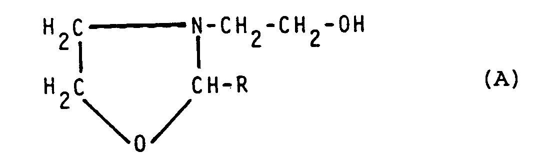 with lower alkyl esters of dicarboxylic or polycarboxylic acids, operating under transesterification conditions; or by means of the reaction of an oxazolidine of formula (B): 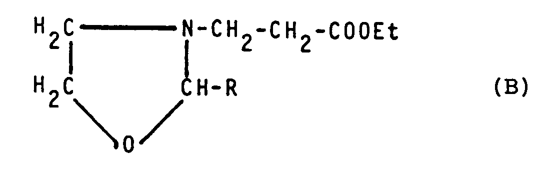 with glycol or a polyol, again operating under transesterification conditions. The oxazolidines (B) may be obtained by the addition of aldehydes to the addition product of ethanolamine and an alkyl acrylate.

As the ester groups can easily be hydrolysed, the polyoxazolidines containing them and also the paints deriving from these acquire a certain instability.

EP-A-228 935 describes the use of polyoxazolidines as cross-linking agents under normal humidity conditions. These polyoxazolidines are derived from bisalkanolamines of formula (C) as starting materials:

HO-CH₂-CH₂-NH-(CH₂)ₙ-NH-CH₂-CH₂-OH (C)

The synthesis of the alkanolamines (C), starting from amines and ethylene oxide, is not very selective. It is also necessary to separate these reaction products from the reaction mass, under conditions of high temperature and vacuum. Distillation is required because of the necessity of eliminating the tertiary amines (tri- and poly-alkanolamines) which, if introduced into the polyisocyanate systems, reduce the pot life of the latter owing to premature cross-linking, both chemically (alkanols) and catalytically (presence of tertiary nitrogen).

US-A-4,296,225 describes the incorporation of polyoxazolidines, at latent cross-linking systems, in polyvinyl systems for the preparation of polyvinyl emulsions. In this case, the oxazolidine is introduced in the form of hydroxyalkyl oxazolidine methacrylate as component of polyurethane paints with a high solids content. The underlying principle is to introduce the oxazolidine nucleus into a polyacrylate, which is made possible by using a vinyl oxazolidine capable of copolymerizing to varying degrees with the acrylic monomers. In all cases the oxazolidine equivalent is not high and the polymers are excessively viscous solids or liquids, making it necessary to disperse them in water or dissolve them in an organic solvent.

IT-A-19089 A/89 describes a new group of polyoxazolidines which can be used as cross-linking agents in systems based on polyisocyanates, acrylates or polyepoxides. The main characteristic of these products is the presence of one or more thioether bridges in the structural formula, which, although imparting stability towards photo-oxidation and a better oil resistance to the manufactured articles into which these products are incorporated, generally have an unpleasant smell, which is typical of polysulphides. In IT-A-20189 A/89 there is described a new group of silane compounds containing at least two oxazolidine groups and a copending Italian patent application describes a new group of polyoxazolidines containing carbonate groups having excellent hydrolytic aging resistance. Finally, in BE-A-833,821, there is described the preparation of polyoxazolidines containing both urethane groups and a combination of isocyanate and urethane groups.

Urethane groups are extremely resistant to hydrolysis, especially in an alkaline medium, and consequently the final paints obtained starting from these compounds are very stable products. A further advantage of the presence of urethane groups in polyoxazolidines is the increased elasticity of the materials produced from them (paints etc.) and their abrasion resistance. The main disadvantage of their use is due to the proposed synthesis which involves the use of aliphatic and/or aromatic polyisocyanates, i.e., products which are notoriously toxic, especially when inhaled.

The present invention relates to a process for the preparation of bisoxazolidines containing urethane groups which overcomes the above drawbacks of the known processes.

In particular, it has been found that it is possible to prepare bisoxazolidines containing urethane groups by using a method which avoids the use of isocyanates and only requires the simple transesterification of the dicarbamate of an aliphatic, cycloaliphatic or aromatic diamine with an N-hydroxyalkyl oxazolidine, said carbamate being obtainable without the use of isocyanates.

Accordingly, the present invention provides a process for the preparation of bisoxazolidines of general formula (I): 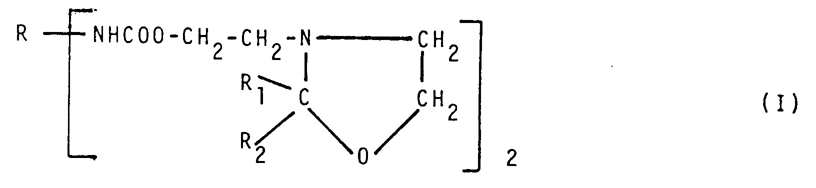 wherein R represents a bivalent group selected from linear or branched C₂-C₁₂ (e.g. C₂-C₆) alkylene, C₇-C₂₀ alkylene-cycloalkylene and alkylene-arylene, C₅-C₈ cycloalkylene and C₆-C₁₄ arylene, said groups, with the exception of alkylene, being optionally substituted by 1, 2, 3 or 4 alkyl groups having 1 to 6, particularly 1 to 4 carbon atoms, and R₁ and R₂, the same or different, represent hydrogen or a linear or branched C₁-C₆ (e.g. C₁-C₄) alkyl group (e.g. methyl or ethyl);
said process comprising the reaction of an N-hydroxyethyloxazolidine of general formula (II) with a dicarbamate of a diamine of general formula (III), e.g. according to the following reaction scheme: 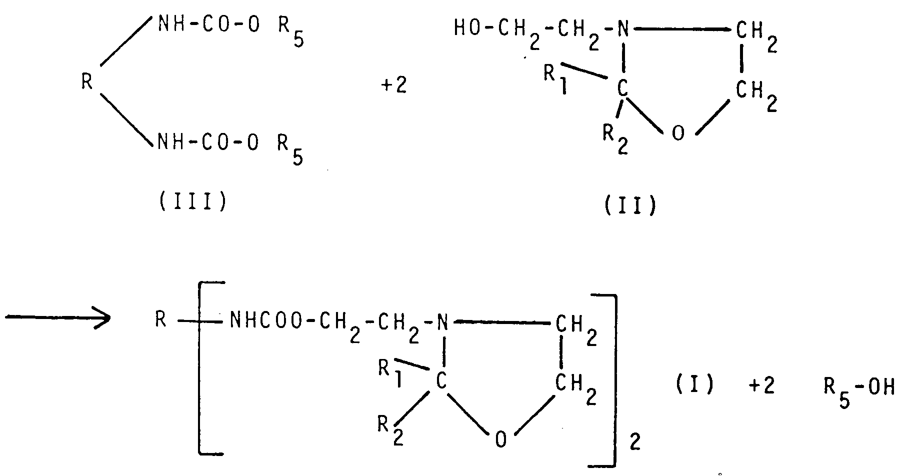 wherein R₅ represents a C₁-C₆ alkyl group; in the presence of a transesterification catalyst selected from sodium, lithium and potassium alcoholates, the reaction being carried out with an up to 50% excess of N-hydroxyethyl oxazolidine (II) and at a temperature ranging from 60 to 140°C and a pressure ranging from atmospheric pressure to 0,1 mmHg such as to allow the removal of the also formed alcohol of general formula R₅OH from the reaction medium by distillation.

Specific examples of bivalent radicals R are the following: (̵CH₂)̵ₘ wherein m is an integer of from 2 to 12 (e.g. 2 to 6),

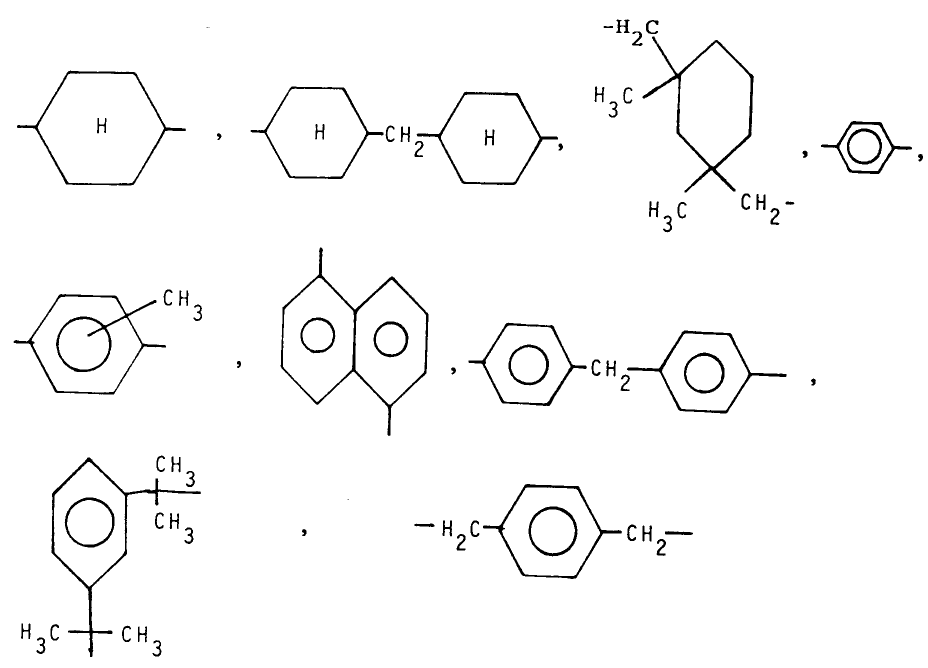

Specific examples of groups R₅ are methyl, ethyl, n-propyl and n-butyl.

More specifically, with reference to the above scheme, a carbamate of general formula (III) is transesterified with a hydroxyethyl oxazolidine of general formula (II), generally in stoichiometric quantities or with a slight excess of (II) with respect to (III) (up to 50%), at a temperature usually ranging from 60 to 140°C (especially 80 to 120°C) and in the presence of a catalyst. The conditions should be such as to allow the alcohol R₅OH to be easily removed by distillation as it is formed. Consequently if R₅ represents a particularly heavy group and therefore the alcohol R₅OH has a particularly high boiling point, it may be necessary to apply a partial vacuum.

In the presence of a suitable catalyst, the reaction between (II) and (III) to give (I) is easily started and may be carried out at a temperature within the above range and with or without a partial vacuum until the stoichiometric quantity of R₅OH has been collected.

The amine carbamate may also be formed in situ without being isolated (see example 4 below) as the catalyst suitable for the formation of the amine carbamate usually is also suitable for the transesterification.

The presence of a solvent is not generally necessary even if in some cases it can be used to facilitate the removal of the alcohol R₅OH during the transesterification. At the end of the reaction and when possible excess quantities of oxazolidine have been removed by distillation under vacuum, the product obtained may be cooled and the catalyst may be eliminated, using the conventional techniques for the catalytic system used.

The excess of oxazolidine ranges from 1 to 50% by moles with respect to the stoichiometric quantity.

As transesterification catalyst a sodium, lithium or potassium alcoholate of a low-boiling (e.g. C₁-C₄) alcohol may be used. The preferred catalyst is sodium methylate.

The quantities of catalyst used usually range from 50 to 1000 ppm with respect to the mixture of reagents.

If sodium methylate is used as catalyst, its removal at the end of the reaction involves, e.g., neutralization with an organic or inorganic acid used in a stoichiometric quantity or a slight excess with respect to the sodium methylate Any possible free acidity is removed by treatment with calcium oxide and subsequent filtration. When the catalyst is sodium methylate, the neutraliser preferably is p-toluene sulfonic acid or phosphoric acid (in a stoichiometric quantity or in a slight excess with respect to the sodium methylate), preferably in the form of concentrated solutions in acetone, methyl ethyl ketone (MEK) or any other suitable solvent.

The polyoxazolidine (I) is obtained in practically quantitative yield.

One advantage of bisoxazolidine structures of the urethane type corresponding to formula (I) as compared to those of the polyester type of the known art is that the former endow the end products with a higher resistance to hydrolysis and a better elasticity and abrasion resistance.

The bicarbamates corresponding to the structural formula (III) are products which are already known in the art, as is their preparation. They are generally prepared by reacting a diamine of formula H₂N-R-NH₂ with a dialkyl or diaryl carbonate in accordance with the following scheme:

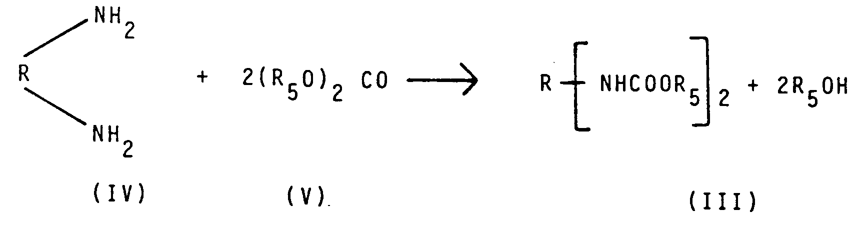

It is preferable to use dimethyl, diethyl, diallyl or diphenylcarbonate in that in the subsequent reaction of (III) with (II) an alcohol or phenol is released then which can easily be removed from the reaction mass.

The reaction between the amine (IV) and the carbonate (V) to give (III) is known; it is fully described, for example, in the following documents:
IT-A-20042 A/89, PCT application WO 8805430, JP-A-02-067261 and US-A-4,097,676, 3,443,019, 3,341,568 and 4,395,565.

The reaction is catalyzed, when alkyl carbonates such as dimethyl or diethyl carbonate are used, by Lewis acids or the alcoholates of alkali or alkaline earth metals. No catalyst is necessary when aryl carbonates such as diphenyl carbonate are used.

For example, hexamethylene dimethylcarbamate,
(CH₂)₆ = (NHCOOR₅)₂, can be obtained by adding an excess of hexamethylene diamine dropwise to dimethyl carbonate at 60 to 70°C under conditions of controlled temperature. This reaction is preferably carried out without a solvent and with about 3% by moles (with respect to the added amine) of sodium methylate as catalyst.

At the end of the reaction, i.e., when the stoichiometric quantity of methyl alcohol has been reached, the catalyst may be decomposed, e.g., by treatment with a stoichiometric quantity of phosphoric acid or p-toluene sulfonic acid or by passing the reaction mixture over an acid resin bed, and the product may be filtered in a boiler, keeping it in its molten state after the excess unreacted dimethyl carbonate has been removed by distillation.

The hexamethylene diamine dicarbamate can easily be purified by recrystallization from the solvent but generally this is not necessary for the purposes of this invention.

For the purposes of the present invention, the term "aryl" preferably refers to mono-, di-, or tricyclic radicals containing from 6 to 14 carbon atoms, optionally substituted.

Similarly the term "arylenic" refers to aromatic bivalent radicals of the same type.

The process of the present invention offers various advantages. First of all, the yields and selectivities of the reactions involved are high. In addition, stable and manageable intermediates are used together with catalysts which can easily be removed; this makes it possible to incorporate the polyoxazolidines into isocyanate systems without prejudicing the pot life.

The polyoxazolidines (I) are compatible with most of the common groups of organic polymers and form latent cross-linking agents in that they hydrolyze immediately in the presence of humidity, even atmospheric humidity, under opening of the oxazolidine ring and generation of polyalkanol amines. They are, therefore, useful as crosslinking agents for, e.g., polyisocyanates, polyepoxides and polyacrylates (Michael addition), particularly in coating, sealant and adhesive compositions. These polyoxazolidines are particularly useful when combined with polyisocyanates in that, owing to their intrinsic characteristics, they do not prejudice the pot life of the latter and can consequently be combined with said polyisocyanates in monocomponent systems which are fluid under normal conditions, preferably without solvents, and are crosslinkable with atmospheric humidity without the formation of foam.

Suitable polyisocyanates for these formulations are those which can be obtained by starting from aliphatic and/or aromatic polyisocyanates and difunctional or polyfunctional low molecular weight (molecular weight of about 350 - 20,000) organic polymers with hydroxy functionalities at the chain-ends of randomly distributed along the chain itself; among these are polyethers, polyesters, polycarbonates, polybutadienes and some hybrid polymers such as copolyether polycarbonate polymers and copolyester polycarbonate polymers with hydroxylic end-groups, and polyacrylates.

These polyisocyanates may be mixed with the polyoxazolidines (I) in such a way that two equivalents of isocyanate groups in the polyisocyanate correspond to one oxazolidine equivalent in the bisoxazolidine. Variations in this stoichiometry are possible without excessively prejudicing the solidity of the cross-linked products, provided that the bisoxazolidine is present in quantities ranging from 30% in defect to 10% in excess with respect to the stoichiometric value.

The formulation of polyisocyanates and polyoxazolidines can be carried out at temperatures ranging from room temperature to 60°C and is facilitated by the perfect compatibility between the two species involved. Suitable catalysts for accelerating the cross-linking may be used in the formulation, normally selected from metallic soaps, and in particular from organometallic compounds of tin, and from organic acids, in particular p-toluene sulfonic acid and benzoic or naphthoic acid. Apart from catalysts, other additives may be incorporated, such as organic or inorganic charges, thixotropic agents, flame-retardants, adhesion promoters, stabilizers, UV absorbers, in accordance with the conventional methods.

The formulations thus obtained cross-link at a high rate, as a result of the atmospheric humidity, to afford end products having excellent general characteristics, especially with respect to thermal and chemical stability and resistance to hydrolytic aging.

The following examples are to further illustrate the present invention without limiting it in any way.

### EXAMPLE 1

Preparation of

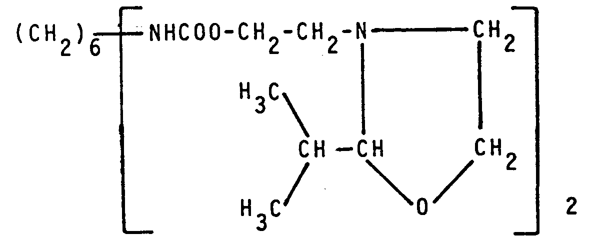

Hexamethylene-1,6-bismethylcarbamate (MW = 232, 348 g, 1.5 moles), N-(2-hydroxyethyl)-2-isopropyl-1,3-oxazolidine (540.8 g, 3.4 moles, and a solution in methanol of sodium methylate (3 ml, 0.9 g of CH₃ONa, 0.0167 moles) were charged, under a nitrogen atmosphere, into a two litre 3-necked flask equipped wiht distillation column), an effective mechanical stirrer and nitrogen inlet.

The flask was immersed in an oil bath and its contents were heated to +110°C, the temperature at which the methanol begins to distill. To facilitate its removal a partial vacuum was applied, which slowly progressed to 50 mmHg and then to 1 mmHg, in accordance with the following table, the temperature in the column being kept at +25°C.

| PRESSURE VARIATION | DURATION | DISTILLED LIQUID |
|---|---|---|
| 1st Phase | 3 hrs | 87 g |
| normal pressure --→50 mmHg | | |
| 2nd Phase | 3 hrs | 16 g |
| 50 mmHg --→ 1 mmHg | | |
| | | $\overline{\text{Total 103 g}}$ |

The analysis of the distillate revealed a 94% concentration of methanol, equal to 96.5 g of alcohol (100% of the theoretical value).

At this stage, the temperature of the boiler was brought to 120°C and that in the column to 110°C. Over a period of 4 hours the excess hydroxyethyl oxazolidine distilled (vacuum of 1 mmHg) (53.6 g; 0.337 moles).

After the product had been cooled to room temperature, p-toluene sulfonic acid was added (3.5 g, 0.0177 moles) in methyl ethyl ketone (10 ml), the addition being carried out dropwise under stirring. After stirring for 30 min, at room temperature, CaO in powder form was added (10 g) and the product was stirred for a further hour.

The product was then filtered under nitrogen, on a G-2 porous glass septum equipped with a celite bed, at a temperature of +75°C.

The product had the following characteristics:
- Yield: 732 g (1.506 moles, 100%)
- Appearance: transparent, homogeneous
- Colour: pale yellow
- Viscosity (+25°C): 2600 cps
- Elemental analysis: C = 58.62%; H = 9.67%; N = 11.23%
[theoretical values for C₂₄H₄₆N₄O₆; MW 486; C = 59.26%; H = 9.47%, N = 11.23%]
NMR spectrum: in accordance with the proposed structure.

### EXAMPLE 2

Preparation of 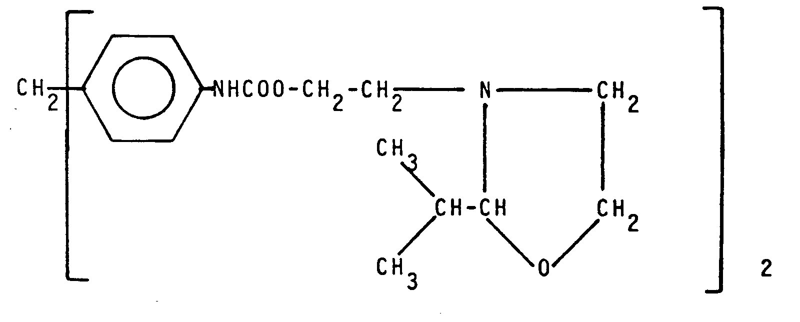 285 g (0.907 moles) of 4,4'-methylene-bis[N-phenyl-O-methylcarbamate] of formula 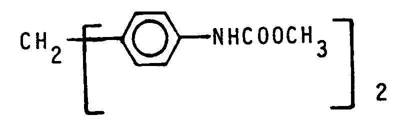 were transesterified with 333.5 g (2.098 moles) of N-(2-hydroxyethyl)-2-isopropyl-1,3-oxazolidine under the same conditions as described in example 1.

The quantity of light distillate (mainly methanol) was 59.9 g, whereas the distilled N-hydroxyethyl-2-isopropyl-1,3-oxazolidine amounted to 39.2 g (70% of the theoretical value).

The product recovered in the flask was practically solid at room temperature, and consequently neutralization was carried out with p-toluene sulfonic acid (3.5 g, 0.0177 moles) in MEK (10 ml) at +50°C and the product was filtered (+110°C) under the same conditions as described in example 1.
- Yield: 518 g (0.907 moles, 100%)
- Appearance: transparent, homogeneous, semi-solid
- Colour: pale yellow
- Viscosity (+50°C): 10,600 cps
- Elemental analysis: C = 65.05%, H = 8.23%, N = 9.70%
(calculated for C₃₁H₄₄N₄O₆: C = 65.49%, H = 7.75%, N = 9.86%; MW = 568)
The NMR spectrum was in accordance with the proposed structure.

### EXAMPLE 3

### Preparation of isophorone-bis-[carbamoyl-2-N-ethyl-2-isopropyl-1,3-oxazolidine]

1,3-isophorone diamine-bis-methylcarbamate (308 g, 1.075 moles) and N-(2-hydroxyethyl)-2-isopropyl-1,3-oxazolidine (396 g, 2.365 moles) were transesterified in the presence of NaOMe (30% by weight in methanol, 6 ml) under the conditions described in example 1.

The light effluents amounted to 62 g (theoretical 68 g, 91%) and were composed of methanol; the heavy effluents, mainly composed (95%) of hydroxyethyl oxazolidine, amounted to 65 g (0.38 moles; theoretical 60.5 g).

The semi-solid product at room temperature was heated to +50°C and neutralized by adding phosphoric acid (3.60 g) in MEK (15 ml) and was finally treated with CaO (10 g).
- Filtration (+100°C): gave 598 g of the product (100% yield).
- Appearance:: limpid, transparent, semi-solid
- Viscosity (+50°C):: 11,000 cps
- Elemental analysis:: C = 61.43%, H = 46%, N = 9.91%
[C₂₈H₅₂N₄O₆ requires C = 62.22%, H = 9.63%, N = 10.37%; MW = 540]
The IR spectrum was in accordance with the given formula.

### EXAMPLE 4

Anhydrous dimethyl carbonate (DMC; 450.25 g, 5 moles), N-(2-hydroxyethyl)-1,3-oxazolidine (334 g, 2.1 moles) and sodium methylate (1.62 g, 5.4 ml of solution in CH₃OH) were charged into a flask equipped with a rectification head, 10-plate column, dropping funnel, thermometer and mechanical stirrer.

The temperature was brought to +65°C and hexamethylene diamine (116.2 g, one mole) was fed from the dropping funnel at such a rate as to keep the temperature at +65°C (1 hour; the reaction was exothermic).

At the end of the addition the reaction mixture was kept at +65°C for a further 2 hours after which the DMC was eliminated under vacuum and the transesterification was subsequently carried out at +65°C for 6 hours, under a maximum vacuum.

The product in the boiler, after neutralization of the sodium methylate with p-toluene sulfonic acid, was treated with CaO and filtered (as in example 1). It had the following characteristics:
- Colour:: pale yellow
- Viscosity (+25°C):: 2700 cps
- Elemental analysis:: C = 58.9%; H = 9.38%; N = 11.32%.

## Claims

1. Process for the preparation of bis-oxazolidines of general formula (I): 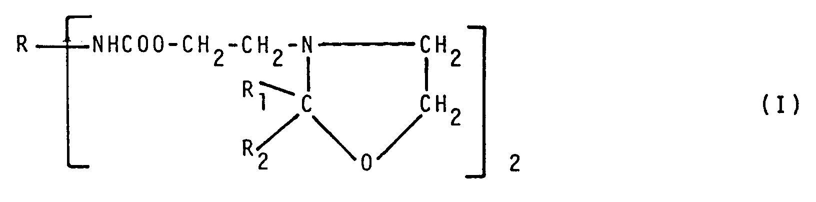 wherein R represents a bivalent group selected from linear or branched C₂-C₁₂ alkylene, C₇-C₂₀ alkylene-cycloalkylene, C₇-C₂₀ arylene-alkylene, C₅-C₈ cycloalkylene and C₆-C₁₄ arylene, said groups, with the exception of alkylene, being optionally substituted by up to 4 C₁-C₆ alkyl groups; R₁ and R₂, the same or different, represent hydrogen or linear or branched C₁-C₆ alkyl;
said process comprising the reaction of an N-hydroxyethyl oxazolidine of general formula (II) with a dicarbamate of general formula (III): 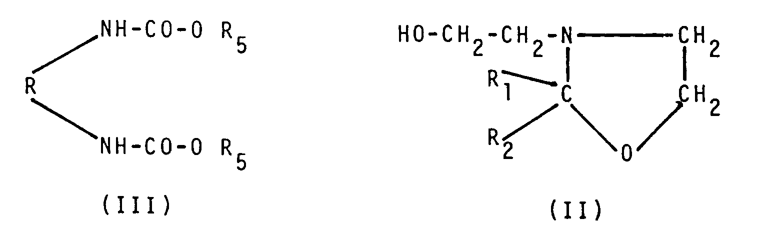 wherein R, R₁ and R₂ have the above mentioned meanings and R₅ represents a C₁-C₆ alkyl group;
in the presence of a transesterification catalyst selected from sodium, lithium and potassium alcoholates, the reaction being carried out with an up to 50% excess of N-hydroxyethyl oxazolidine (II) and at a temperature ranging from 60 to 140°C and a pressure ranging from atmospheric pressure to 0,1 mmHg such as to allow the removal of the also formed alcohol of general formula R₅OH from the reaction medium by distillation.

2. Process according to claim 1, wherein the transesterification temperature ranges from 80 to 120°C.

3. Process according to any one of the preceding claims, wherein the transesterification reaction is carried out in the presence of a solvent.

4. Process according to any one of the preceding claims, wherein the amount of catalyst used ranges from 50 to 1000 ppm with respect to the mixture of reagents.

## Patentansprüche

1. Verfahren zur Herstellung von Bisoxazolidinen der allgemeinen Formel (I): 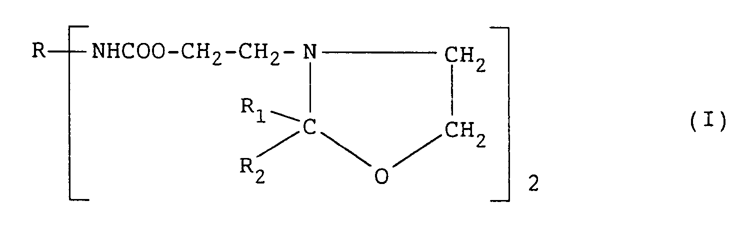 worin R eine bivalente Gruppe ausgewählt aus linearem oder verzweigtem C₂-C₁₂-Alkylen, C₇-C₂₀-Alkylen-cycloalkylen, C₇-C₂₀-Arylen-alkylen, C₅-C₈-Cycloalkylen und C₆-C₁₄-Arylen bedeutet, wobei diese Gruppen, mit Ausnahme von Alkylen, gegebenenfalls mit bis zu 4 C₁-C₆-Alkylgruppen substituiert sein können; R₁ und R₂, die gleich oder verschieden sein können, Wasserstoff oder lineares oder verzweigtes C₁-C₆-Alkyl bedeuten;
welches Verfahren die Umsetzung eines N-Hydroxyethyloxazolidins der allgemeinen Formel (II) mit einem Dicarbamat der allgemeinen Formel (III): 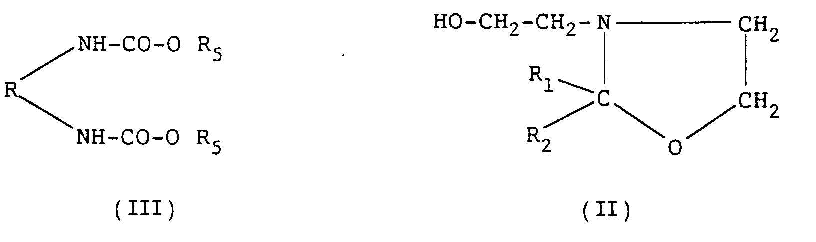 worin R, R₁ und R₂ die oben genannte Bedeutung haben und R₅ eine C₁-C₆-Alkylgruppe bedeutet; in Gegenwart eines Umesterungskatalysators, ausgewählt aus Natrium-, Lithium- und Kaliumalkoholaten, umfaßt, wobei die Umsetzung mit einem bis zu 50%igen Überschuß an N-Hydroxyethyloxazolidin (II) und bei einer Temperatur im Bereich von 60 bis 140°C und einem Druck im Bereich von Atmosphärendruck bis 0,1 mmHg erfolgt, so daß sich der ebenfalls gebildete Alkohol der allgemeinen Formel R₅OH aus dem Reaktionsmedium durch Destillation entfernen läßt.

2. Verfahren nach Anspruch 1, worin die Temperatur für die Umesterung im Bereich von 80 bis 120°C liegt.

3. Verfahren nach irgendeinem der vorhergehenden Ansprüche, worin die Umesterungsreaktion in Gegenwart eines Lösungsmittels durchgeführt wird.

4. Verfahren nach irgendeinem der vorhergehenden Ansprüche, worin die eingesetzte Menge Katalysator im Bereich von 50 bis 1000 ppm liegt, bezogen auf die Mischung der Reagenzien.

## Revendications

1. Procédé de préparation de bis-oxazolidines de la formule générale (I): 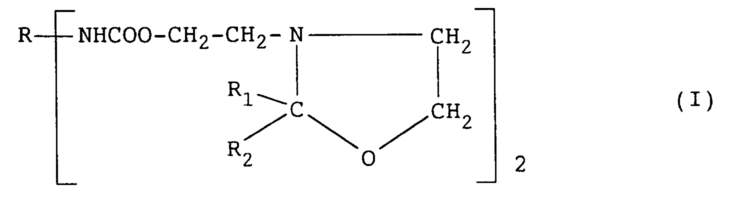 dans laquelle R représente un groupe bivalent sélectionné d'alkylène C₂-C₁₂ ramifié ou linéaire, d'alkylène-cycloalkylène C₇-C₂₀, d'arylène-alkylène C₇-C₂₀, de cycloalkylène C₅-C₈ et d'arylène C₆-C₁₄, lesdits groupes à l'exception d'alkylène étant d'une manière facultative substitués par jusqu'à 4 groupes alkyle C₁-C₆; R₁ et R₂, identique ou différent, représentent de l'alkyle C₁-C₆ ramifié ou linéaire ou hydrogène;
ledit procédé comprenant la réaction de N-hydroxyéthyle oxazolidine de la formule générale (II) avec un dicarbamate de la formule générale (III) 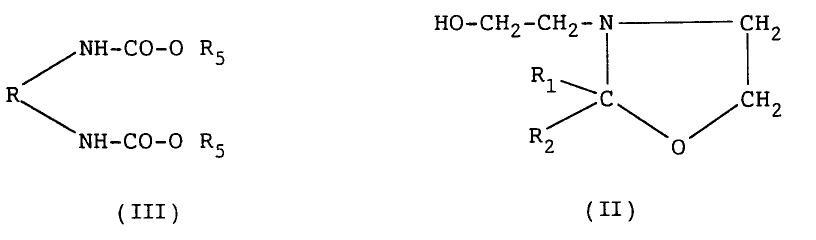 dans lesquelles R, R₁ et R₂ ont les significations mentionnées ci-dessus et R₅ représente un groupe alkyle C₁-C₆; en présence d'un catalyseur de transestérification sélectionné des alcoolats de sodium, lithium et potassium, la réaction étant effectuée avec un excédent allant jusqu'à 50% de N-hydroxyéthyle oxazolidine (II) et à une température variant de 60 à 140°C et à une pression variant de la pression atmosphérique à 0,1 mmHg afin de permettre l'enlèvement de l'alcool également formé de la formule générale R₅OH du milieu de réaction par distillation.

2. Procédé selon la revendication 1, dans lequel la température de transestérification varie de 80 à 120°C.

3. Procédé selon l'une quelconque des revendications précédentes, dans lequel on effectue la réaction de transestérification en présence d'un solvant.

4. Procédé selon l'une des revendications précédentes, dans lequel la quantité du catalyseur utilisé varie de 50 à 1000 ppm par rapport à la mixture de réactifs.
